# EUROPEAN PATENT APPLICATION

(11) **EP 1 731 104 A1**
(43) Date of publication of application: **13.12.2006**
(21) Application number: 06010579.8
(22) Date of filing: 23.05.2006
(51) Int. Cl.: A61B 10/02

(54) **Bayonet release of safety shield for needle tip**

(30) Priority: 06.06.2005 US 146173
(71) Applicant: Sherwood Services AG, 8200 Schaffhausen (CH)
(72) Inventor: Swisher, David Rork, St. Charles MO 63301 (US); Moos, Kimberly A., Florissant MO 63033 (US); Reynolds, Whitney, Cumberland RI 02864 (US)
(74) Representative: Tomlinson, Edward James

(57) **Abstract**

A needle assembly having a shield attached for quick release from the remainder of the needle assembly to be moved to cover the sharp tip. The shield can be disconnected by rotation of only about 90 degrees. When not needed to cover the sharp tip, the shield is held in a stowed position out of the way so as not to interfere with use of the needle assembly.

## Description

### CROSS REFERENCE TO RELATED APPLICATION

This application includes subject matter in common with co-assigned U.S. Patent application Serial No. 11/145,684 entitled Needle Assembly with Removable Depth Stop, filed on June 6, 2005. The subject matter of this application is incorporated herein in its entirety by reference.

### BACKGROUND OF THE INVENTION

This invention relates generally to needle assemblies and more particularly to needle assemblies that have shields to cover sharp ends of needles.

Needle assemblies of the present invention have particular, although not exclusive application in the field of medicine and have needles with sharpened ends for use in piercing the skin to inject or withdraw materials as needed. The needle assembly may also be used to injector withdraw material from an IV line so that penetration of the skin is not always undertaken.

The needle is supported by some other structure that is used to manipulate the needle. The most common example is a syringe. However, some needle assemblies require the application of substantial force in use. One example of such a needle assembly is a bone marrow needle assembly that is used to penetrate cortical bone to reach the intramedullary canal for withdrawing liquid or a biopsy sample of bore marrow, or for infusing the canal with a selected material. Typically, the needle includes a cannula and a stylet that is received in the cannula and has a hard, sharp tip that can penetrate cortical bone. The tip projects out from the distal end of the cannula. The stylet can be withdrawn from the cannula after the needle penetrates the bone to the so that the hollow interior of the cannula can be used as a conduit for liquid or a receptacle to collect bone marrow.

In order to penetrate cortical bone, a substantial amount of force must be applied to the needle. For this reason, bone needle assemblies conventionally mount the needle in a handle that is sized and shaped so that the technician may comfortably grip the handle and apply the force necessary to penetrate the bone. The handle may comprise two handle members that can be selectively put together and separated for inserting the stylet into the cannula and removing the stylet from the cannula. A proximal handle member mounts the stylet and a distal handle member mounts the cannula. "Proximal" and "distal" refer to the relative location of the handle members to the technician when the needle assembly is in use. The proximal handle member is in contact with the palm of the technician's hand in use, and the distal handle member is on the opposite side of the proximal handle member from the palm.

Some needle assemblies, including bone needle assemblies, have associated safety mechanisms that shield the sharp tips of the needle components when they are not needed and after they have become contaminated with potentially hazardous biological material. The safety mechanism includes a shield and usually a mechanism for locking the shield in place over the sharpened tip. As a matter of convenience, and to enhance the probability that the safety feature will be used by a medical technician, the safety feature may be secured to the needle assembly. However, the safety feature must be retained out of the way when the needle assembly is being used, for example, to collect a liquid or solid sample from the intramedullary canal. The safety feature then must be released from its stowed position and moved to an operative position in which its shield covers the sharpened tip of the needle. The operation of the safety feature needs to be quick and easy for the medical technician who has many other tasks and other instruments that require specialized knowledge to use. Failure to make the operation of the safety device rapid and clear to the user may result in the feature not being used at all, thereby defeating the purpose of the safety feature.

### SUMMARY OF THE INVENTION

In one aspect of the present invention, a needle assembly generally comprises a handle for manipulating the needle assembly, and a needle mounted on the handle and extending outwardly therefrom. The needle has a longitudinal axis and a sharp end. A safety shield associated with the needle can move relative to the needle between a stowed position in which the safety shield is spaced from the sharp end of the needle and a deployed position in which the safety shield covers the sharp end. A bayonet connector is adapted to connect the safety shield to the handle in the stowed position and to quickly release the connection of the safety shield to the handle to permit movement of the safety shield to the deployed position covering the sharp end of the needle.

In another aspect of the present invention, a needle assembly generally comprises mounting structure, a needle and a safety shield as set forth previously. The needle assembly further includes a rotary connector adapted to connect the safety shield to the handle in the stowed position and to quickly release the connection of the safety shield to the mounting structure by rotation of the connector about the longitudinal axis of the needle less than 360 degrees to permit movement of the safety shield to the deployed position covering the sharp end of the needle.

In yet another aspect of the present invention, a method of shielding a sharp end of a needle of a needle assembly generally comprises rotating a connector attaching a safety shield to the needle assembly about an axis of the needle less than a full turn thereby to release the connection of the safety shield to the needle assembly. The safety shield is slid along a longitudinal axis of the needle to a position in which the safety shield covers the sharp end of the needle.

Other objects and features of the present invention will be in part apparent and in part pointed out hereinafter.

### BRIEF DESCRIPTION OF THE DRAWINGS

FIG. 1 is a perspective of a bone needle assembly;

FIG. 2 is the perspective of Fig. 1 with a safety shield of the assembly rotated to a disconnected position;

FIG. 3 is a perspective of the bone needle assembly showing a proximal handle member and stylet partially separated from a distal handle member and cannula;

FIG. 4 is the perspective of Fig. 3 rotated so as to be seen from the opposite side;

FIG. 5 is the perspective of Fig 2 with the safety shield moved to a position covering a sharp end of a needle of the assembly;

FIG. 6 is an elevation of the safety shield;

FIG. 6A is a fragmentary elevation of the safety shield taken from the vantage indicated by line 6A-6A in Fig. 6;

FIG. 7 is an elevation of a distal handle member of the assembly;

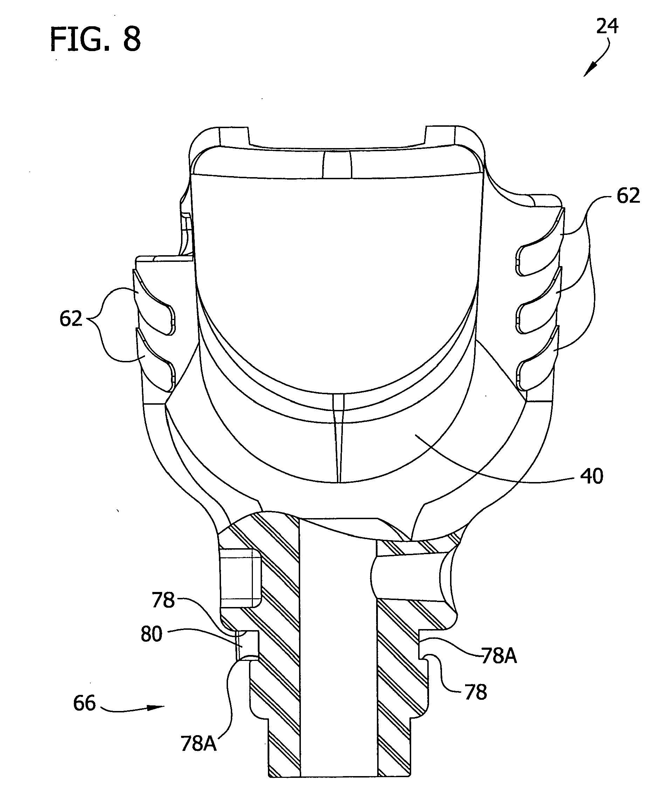

FIG. 8 is a left side elevation of the distal handle member with portions broken away to show details of construction;

FIG. 9 is aright side elevation of the distal handle member and safety shield with portions broken away and showing the safety shield connected to the distal handle member; and

FIG. 10 is an enlarged fragmentary portion of the perspective of Fig. 5 showing the safety shield with portions broken away to show internal construction.

Corresponding reference characters indicate corresponding parts throughout the several views of the drawings.

### DETAILED DESCRIPTION

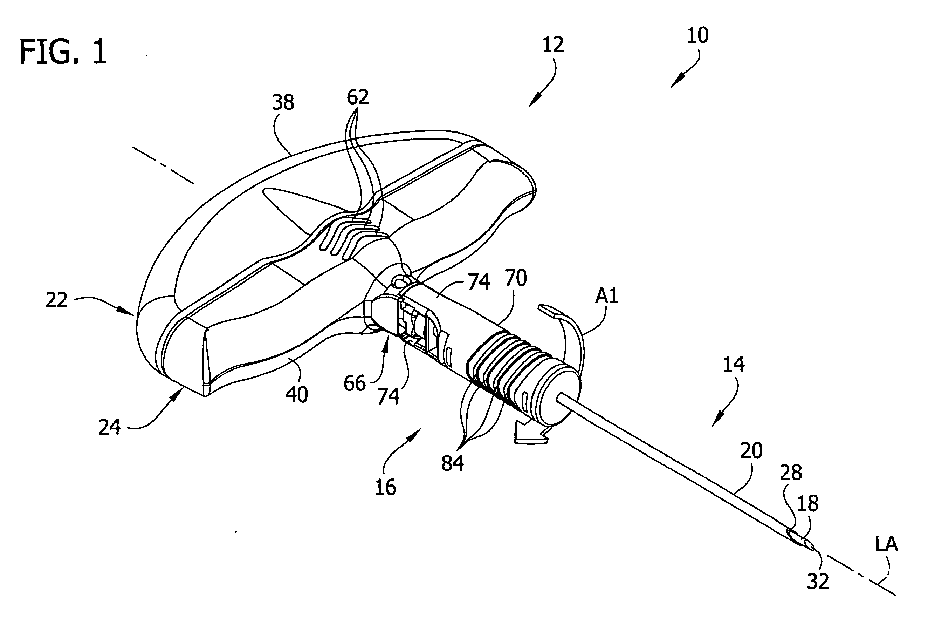

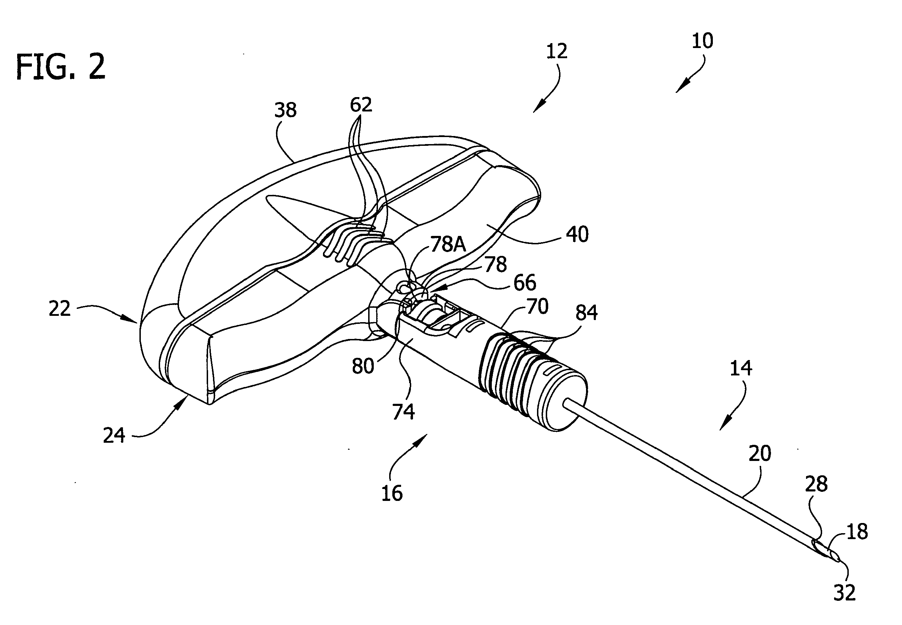

Referring now to the drawings and in particular to Figs. 1 and 2, a medical instrument constructed according to the principles of the present invention is shown in the form of a bone needle assembly, generally indicated at 10. The bone needle assembly includes a handle 12 (broadly, "mounting structure"), a needle 14 and a cannula safety shield 16 (broadly, "an operative member"), all reference numbers indicating their subjects generally. The needle 14 includes a stylet 18 and a cannula 20 that can receive the stylet. The handle 12 includes a first or proximal handle member (indicated generally at 22) mounting the stylet 18, and a second or distal handle member (indicated generally at 24) mounting the cannula 20. It will be understood that a needle could include only a single component part, or more than two parts within the scope of the present invention. Similarly, a handle could be a single part or more than two parts. The mounting structure for the needle 14 can be other than a handle without departing from the present invention. Still further, the operative member could be other than a shield, and the needle 14 could be omitted without departing from the scope of the present invention.

The cannula 20 has an axial passage extending the length of the cannula and opening at both ends of the cannula. A distal tip 28 of the cannula 20 is beveled and sharpened. A proximal end portion of the cannula 20 is received in the distal handle member 24. The stylet 18 is solid and includes a sharp distal tip 32, and a proximal end portion received in the proximal handle member 22. The stylet 18 can be inserted through the axial passage opening in the proximal end portion of the cannula 20 and received entirely through the axial passage of the cannula so that its sharp distal tip 32 projects axially outward from the distal tip 28 of the cannula. The stylet 18 provides the tool for penetrating the cortical bone, and can be removed from the cannula 20 once the intramedullary canal is accessed by the needle 14.

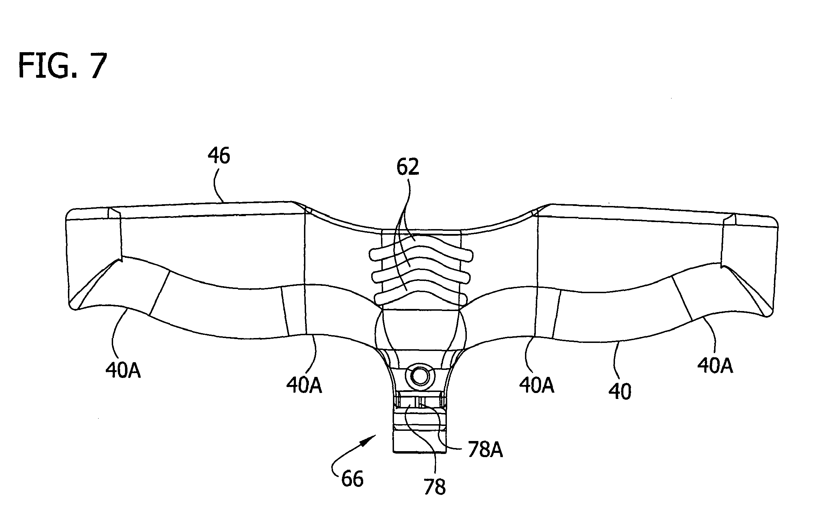

The handle 12 formed by the proximal and distal handle members 22, 24 has an ergonomic shape that can be comfortably received in a medical technician's hand, and allows the technician to easily control the needle assembly 10 as he or she applies the substantial forces needed to penetrate the bone. More specifically, the top or proximal surface 38 of the proximal handle member 22 is rounded in conformance with the shape of the palm of the hand. The bottom or distal surface 40 of the distal handle member 24 is also rounded, but is undulating in shape thereby forming finger wells 40A for receiving the technician's fingers (see, Fig. 7). The proximal and distal handle members 22, 24 can be securely connected together when the stylet 18 is received in the cannula 20, so that the handle 12 acts essentially as a single piece when used to drive the needle 14 through a patient's skin and into the bone. The proximal and distal handle members 22, 24 can be disconnected and moved apart for removing the stylet 18 from the cannula 20.

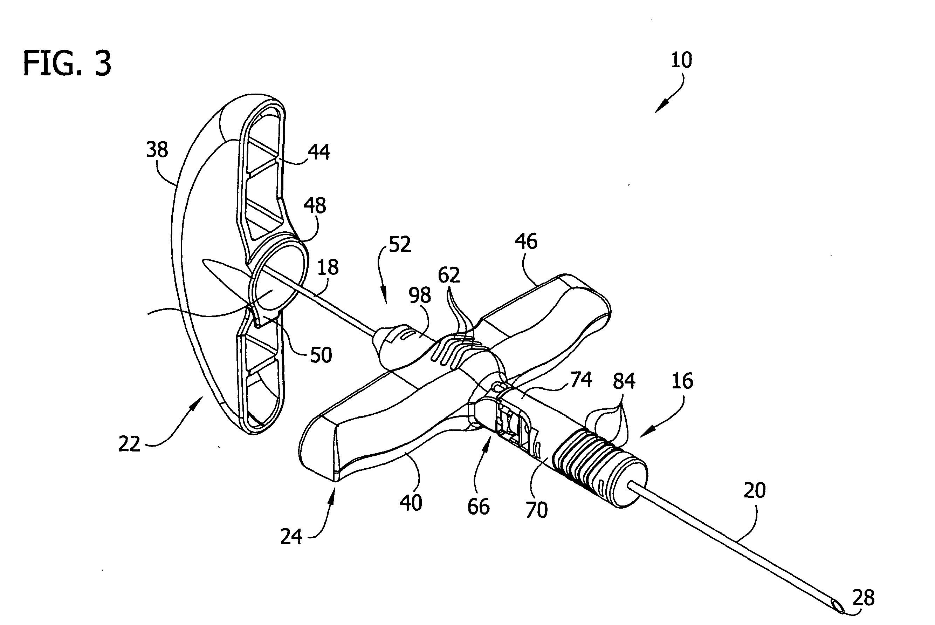

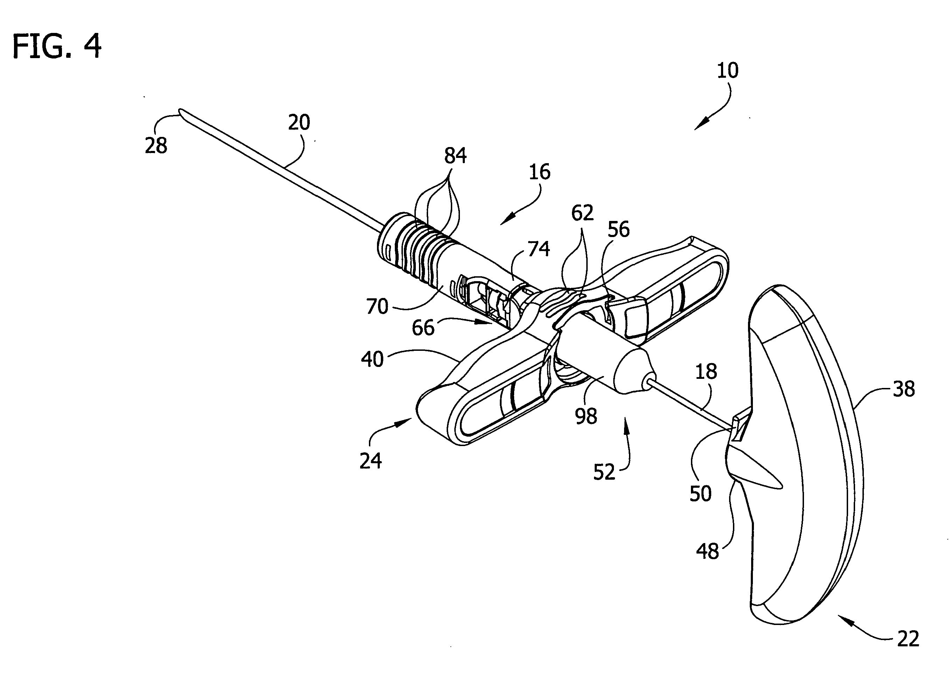

The proximal handle member 22 has a distal surface 44 and the distal handle member 24 has a proximal surface 46 that are formed to mate in flush engagement with each other when the handle members are engaged. The proximal handle member 22 includes a central annular mounting portion 48 having a tab 50 projecting laterally from one side of the mounting portion (Figs. 3 and 4). The distal handle member 24 has an open central portion on its proximal surface 46 that can partially receive the central mounting portion 48 and a stylet safety shield 52. The operation this shield 52 will be described hereinafter. The distal handle member 24 is formed with a groove 56 extending along an arc in a direction around a longitudinal axis of the cannula 20 and opening on one side of the distal handle member (Fig. 4). The shield 52 remains with the distal handle member 24 before the proximal handle member 22 is completely separated from the distal handle member (as is shown in Fig. 3).

To assemble the proximal handle member 22 and stylet 18 with the distal handle member 24 and cannula 20, the sharp distal tip 32 of the stylet is aligned with a central passage of the distal handle member that communicates with the axial passage of the cannula 20, and inserted into the central passage. The stylet 18 can be inserted further so that it enters the axial passage of the cannula 20. This stage of assembly is illustrated in Figs. 3 and 4. The proximal and distal handle members are turned from their aligned position shown in Figs. 1 and 2 to a position in which the proximal handle member 22 is perpendicular to the distal handle member 24. When the handle members are fully brought together, the tab 50 on the proximal handle member 22 is generally aligned with the groove 56 on the distal handle member 24. By turning the handle members toward alignment with each other, the tab 50 is received in the groove 56 thereby securing the proximal handle member 22 to the distal handle member 24. Thereafter, it will require nearly a 90 degree turn of the proximal handle member 22 relative to the distal handle member 24 to disconnect these components. Accordingly, accidental separation of the handle members 22, 24 in use is resisted. Wavy ribs 62 on the distal handle member 24 are provided for gripping the distal handle member to disconnect and separate the distal handle member from the proximal handle member 22. As illustrated, there are three ribs 62 on one side of the distal handle member 24 (Fig. 3) and two ribs on the opposite side (Fig. 4). The ribs could be provided in a different number or omitted altogether without departing from the scope of the present invention. The wave shape of the ribs 62 suggests that first twisting and then axial movement is needed to achieve separation of the proximal handle member 22 and stylet 18 from the distal handle member 24 and cannula 20.

A proximal end portion of the stylet 18 extends through the stylet safety shield 52 into the open center of the proximal handle member 22. A top end of the stylet 18 is secured is a suitable manner to the proximal handle member 22. For instance, the handle member 22 may be molded around the stylet 18 or the stylet attached to the proximal handle member by an adhesive. The proximal handle member 22 can be formed of polymeric or other material. Although shown as opaque in the drawings, the proximal handle member 22 could be partially or totally transparent. A proximal end portion of the cannula 20 extends into a tubular, projecting portion of the distal handle member 24 (indicated generally at 66) located at its center. The cannula 20 is mounted on the distal handle member 24 in a suitable manner. For instance, the distal handle member 24 may be molded around the cannula 20 or the cannula may be adhered to the distal handle member. The distal handle member 24 can be formed of polymeric or other suitable material. Although shown as opaque, the handle member 24 could be partially or totally transparent.

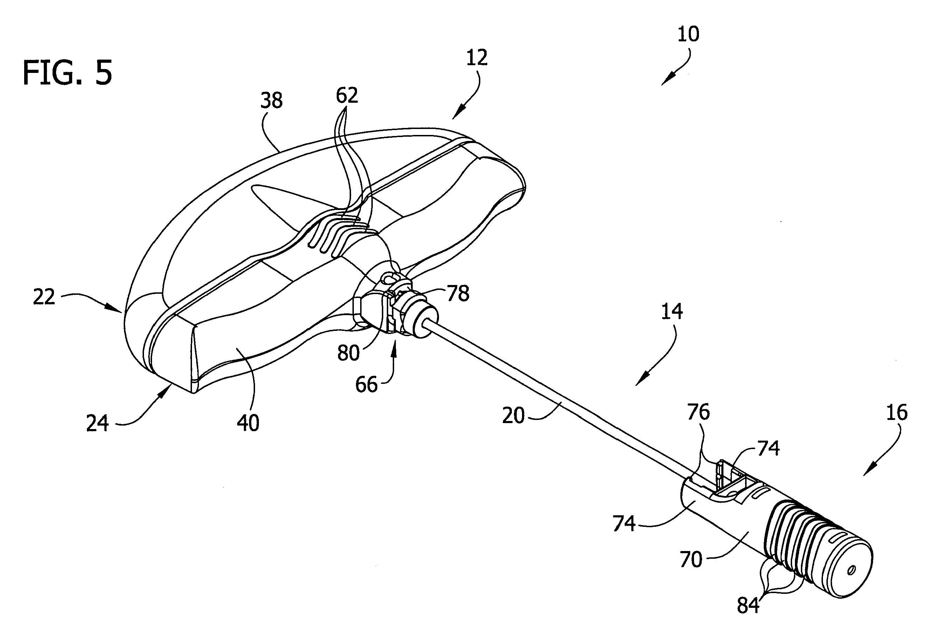

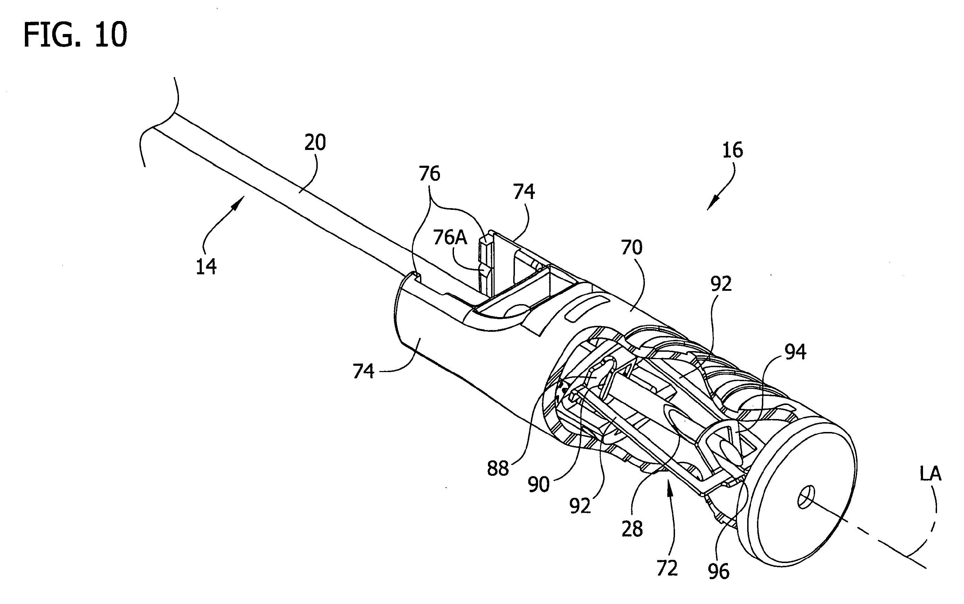

The cannula safety shield 16 is shown in Figs. 5 and 10 extended to cover the distal tip 28 of the cannula 20 after the needle assembly 10 has been used. The safety shield 16 includes a generally tubular housing 70 and an internal locking mechanism (generally indicated at 72 in Fig. 10) capable of releasably locking the tubular housing in position covering the distal tip 28 of the cannula 20. The tubular housing 70 includes a pair of opposite, axially projecting arms 74 at a proximal end of the housing. The free ends of the arms 74 have radially inwardly projecting fingers 76 that are capable of being received in respective arcuate slots 78 formed on the exterior of the tubular portion 74 of the distal handle member 24. The slots 78 each extend generally along an arc about a longitudinal axis LA of the needle 14. In the illustrated embodiment, the arms 74 and fingers 76 constitute a "rotary connector" and/or "a bayonet connector." It will be understood that a rotary connector or bayonet connector may take on other forms within the scope of the present invention.

The fingers 76 and slots 78 cooperate to secure the safety shield 16 to the distal handle member 24 in a stowed position when not needed (e.g., as shown in Fig. 1). By turning the tubular housing 70 about ninety degrees relative to the distal handle member 24 about a longitudinal axis LA of the needle 14 as indicated by arrow A1 in Fig. 1, the fingers 76 can be removed from the slots 78 so that the safety shield 16 is released from connection to the distal handle member (Fig. 2). Although the tubular housing 70, arms 74 and fingers 76 are shown as being formed of one piece of material, a "connector" (e.g., the arms and fingers) may be formed separately from the remainder of a safety shield. Moreover, the connector (e.g., arms 74 and fingers 76) may move independently of the tubular housing 70 without departing from the scope of the present invention. The slots 78 each are open on one circumferential end of the slot and include an end wall 80 on the other end of the slot. The end wall 80 locates the fingers 76 and positions the safety shield 16 relative to the handle 12.

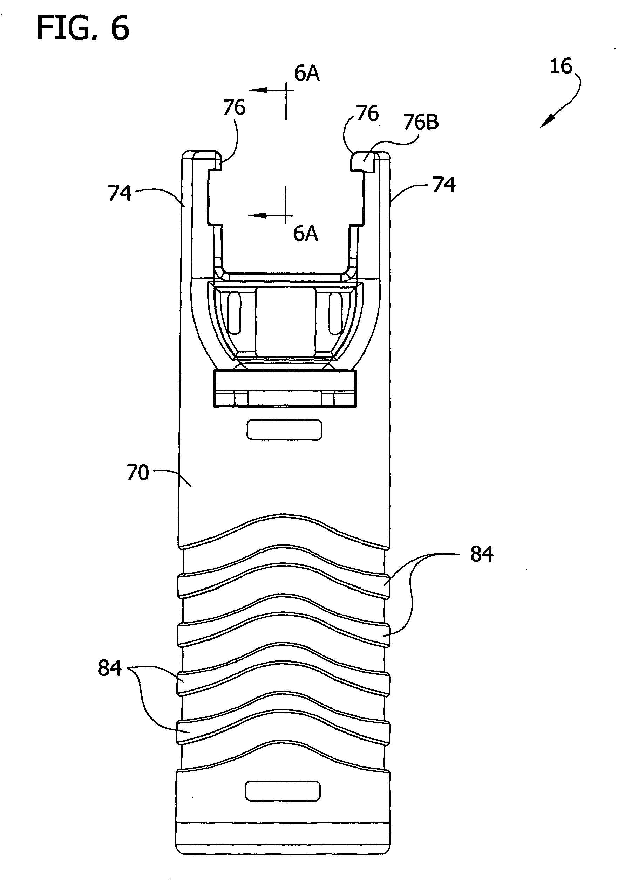

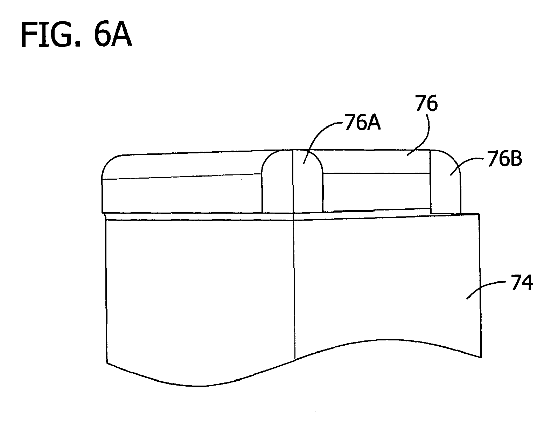

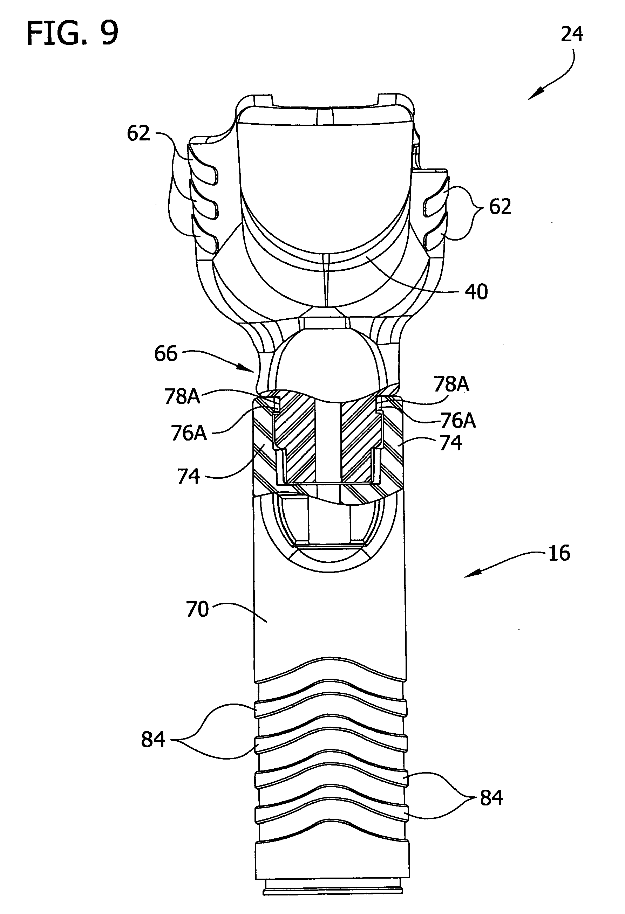

The fingers 76 and slots 78 are constructed so that they retain their connection. More specifically, each of the fingers 76 each have a generally triangular shaped recess 76A located generally in the middle of the finger (see Fig. 6A) that are sized to receive a triangular projection 78A located generally in the center of each slot 78 (e.g., see Fig. 7). When the triangular projections 76A are received in the triangular recesses 78A, the safety shield 16 is held against rotation relative to the tubular portion 66 of the distal handle member 24 (Figs. 1 and 9). Thus, the safety shield 16 is prevented from inadvertent disconnection from the distal handle member 24. By applying sufficient force, the interlocked connection of the triangular recesses 76A and triangular projections 78A can be overcome to release the safety shield 16 from the distal handle member 24. To connect the safety shield 16 to the distal handle member 24, the safety shield is rotated in a direction opposite arrow A1 from its Fig. 2 position back to its Fig. 1 position. A tapered leading edge portion 76B of each finger 76 first enters its respective slot 78 and eventually engages the triangular projection 78A. The tapered shape of the leading edge portion 76B allows each arm 74 to be resiliently deflected by a small amount in a radially outward direction with respect to the longitudinal axis LA of the needle 14. As the safety shield 16 continues to be rotated, each triangular recess 76A is eventually brought into registration with the corresponding triangular projection 78A. The resilience of the material of the arms 74 forces the recesses 76A down onto the triangular projections 78A so that the projections are partially received in the recesses to retain the connection. The technician will experience a tactile or audible snap as a result of this registering event that confirms the safety shield 16 is secured in place. By applying sufficient torque in the direction of arrow A1, the fingers 76 can rotate to move the triangular recesses 76A off of the triangular projections 78A through deflection of the arms 78. It will be understood that the shape of a projection and recess may be other than triangular. Moreover, the projection could be on a finger and a recess could be in a slot of a handle. Still further, the retention feature could be omitted within the scope of the invention.

The connection of the safety shield 16 with the tubular portion 66 of the distal handle member may be described as "bayonet". However, it will be understood that other types of connections may be used within the scope of the present invention. Generally speaking, a quick release connection is desirable. For rotary connections, the fingers 76 should preferably enter the slots 78 and engage the end wall 80 with less than a 360 degree turn of the connector. Connection can be made by turning the safety shield 16 from the position in Fig. 2 back to the position of Fig. 1 in a direction opposite that indicated by arrow A1. Release of the fingers 76 from a position at the end walls 80 in the slots 78 should occur with the same rotation, just in the opposite direction. More preferably, the amount of rotation necessary to engage and/or release the fingers 76 is less than 180 degrees to release connection. The bayonet connection illustrated in the drawings requires only about a 90 degree turn to achieve both connection and disconnection.

Once the safety shield 16 is disconnected from the distal handle member 24 by this motion, the safety shield can freely slide down the needle 14 to a deployed position covering the sharp distal tips 28, 32 of the cannula 20 and stylet 18, as is illustrated in Fig. 5. The locking mechanism 72 automatically secures the safety shield 16 in place covering the sharp distal tip 28 of the cannula 20. Although the tubular housing 70 of the safety shield 16 is shown as being opaque, it may be totally or partially transparent to visible electromagnetic radiation so that activation of the locking mechanism 72 could be confirmed by looking through the tubular housing. If desired, the safety shield 16 can be reattached to the needle 14 because the disconnection is non-destructive.

The tubular housing 70 is formed with ribs 84 that extend generally circumferentially around the housing, as best seen in Fig. 6. The ribs 84 have a generally wavy formation as they extend around the circumference of the housing 70. The wavy formation, which extends both axially and circumferentially is provided to show the medical technician that the way to operate the safety shield 16 is to turn the housing 70 in a direction around its circumference and then slide the safety shield axially down to the end of the cannula 20.

The locking mechanism 72 inside the safety shield 16 comprises a canting member including a base 88 having a hole 90 and a pair of arms 92 extending generally axially from the base. The arms are connected together by a U-shaped member 94 at their ends and each has an upwardly (as oriented in the figures) bent tab 96 projecting axially outward from the end. Before the locking mechanism 72 is activated to lock the tubular housing 70 in position, the ends of the arms 92 ride on the exterior surface of the cannula 20. This holds the canting member so that the base 88 is orthogonal so the longitudinal axis of the cannula 20 and the base can move along the cannula (with the safety shield 16), with the cannula sliding unimpeded through the hole 90 in the base. Once the ends of the arms 92 pass the distal tip 28 of the cannula 20, the locking mechanism 70 is weighted so that the ends of the arms move in a generally radial direction toward an opposite side of the longitudinal axis LA of the needle 14. This causes the base 88 of the canting member to cant relative to the axis of the needle 14 so that the hole 90 in the base is no longer orthogonal to the axis of the cannula. As a result, the base 88 at the edge of the hole 90 grippingly engages the cannula 20 to lock the safety shield 16 in place. It will be understood that a locking mechanism could be omitted or take on other forms than shown and described without departing from the scope of the present invention.

The stylet safety shield 52 (Figs. 3 and 4) has a similar construction and operation as the cannula safety shield 16. The stylet safety shield 52 includes a tubular housing 98 that can be releasably secured (as by an interference fit) to the distal handle member 24 inside the central opening. As the stylet 18 and proximal handle member 22 are pulled out of the cannula 20, the tubular housing 98 of the safety shield 52 remains held in the central opening of the distal handle member 24 so that the stylet slides through the tubular housing. When the sharp end 32 of the stylet 18 exits the distal handle member 24 and moves into the tubular housing 98, a locking mechanism (which may be of substantially the same construction and operation as the locking mechanism 72 of the cannula safety shield 16) is activated to grip the stylet. Thereafter, the safety shield 52 is held on the stylet 18 in a position covering the sharp end 32. Continued movement of the proximal handle member 22 and stylet 18 away from the distal handle member 24 and cannula 20 releases the tubular housing 98 from the central opening of the distal handle member so that the safety shield 52 separates from the distal handle member and travels with the stylet. The tubular housing 98 of the stylet safety shield 52 is shown as opaque, but could be totally or partially transparent to visible electromagnetic radiation so that activation of the locking mechanism can be visually confirmed.

When introducing elements of the present invention or the preferred embodiment(s) thereof, the articles "a", "an", "the" and "said" are intended to mean that there are one or more of the elements. The terms "comprising", "including" and "having" are intended to be inclusive and mean that there may be additional elements other than the listed elements. Moreover, the use of "up", "down", "top" and "bottom" and variations of these terms is made for convenience, but does not require any particular orientation of the components.

As various changes could be made in the above without departing from the scope of the invention, it is intended that all matter contained in the above description and shown in the accompanying drawings shall be interpreted as illustrative and not in a limiting sense.

## Claims

1. A needle assembly (10) comprising:
a mounting structure (12);
a needle (14) mounted on the mounting structure and extending outwardly therefrom, the needle having a longitudinal axis (LA) and a sharp end (28, 32);
a safety shield (16) associated with the needle for movement relative to the needle between a stowed position in which the safety shield is spaced from the sharp end of the needle and a deployed position in which the safety shield covers the sharp end;
a rotary connector (74, 76) adapted to connect the safety shield (16) to the mounting structure (12) in the stowed position and to quickly release the connection of the safety shield to the mounting structure by rotation of the rotary connector about the longitudinal axis of the needle less than 360 degrees to permit movement of the safety shield to the deployed position covering the sharp end of the needle.

2. A needle assembly as set forth in claim 1 wherein the rotary connector (74, 76) is constructed to connect to the mounting structure (12) and release from the mounting structure by making turns that are less than 180 degrees.

3. A needle assembly as set forth in claim 2 wherein the rotary connector (74, 76) is constructed to connect to the mounting structure (12) and release from the mounting structure by making turns that are about 90 degrees.

4. A needle assembly as set forth in any one of claims 1 to 3 wherein the rotary connector comprises a finger (76) and the mounting structure includes a slot (78), the finger being sized and shaped for reception in the slot to connect the safety shield (16) to the mounting structure (12).

5. A needle assembly as set forth in claim 4 wherein the slot (78) is formed to retain the finger (76) therein.

6. A needle assembly as set forth in claim 5 wherein the slot (78) extends along an arc generally about the longitudinal axis (LA) of the needle (14).

7. A needle assembly as set forth in claim 6 wherein the rotary connector further comprises a pair of arms (74) extending from the safety shield (16) and a finger (76) on a free end of each arm, the mounting structure (12) including a slot (78) for each finger sized and shaped to receive the finger.

8. A needle assembly as set forth in claim 1 wherein the rotary connector and the safety shield are formed as one piece.

9. A medical instrument (10) comprising:
mounting structure (12);
an operative member (16) associated with the mounting structure for movement relative to the mounting structure between a first position and a second position spaced from the first position;
a rotary connector (74, 76) adapted to connect the operative member (16) to the mounting structure (12) in the first position and to quickly release the connection of the operative member to the mounting structure by rotation of the rotary connector about the longitudinal axis (LA) of the needle, at least one of the rotary connector and mounting structure including a retention structure (76A, 78A) adapted to interengage the rotary connector and mounting structure in the first position to resist relative rotation of the rotary connector and mounting structure for securing the operative member against inadvertent release from the mounting structure.

10. A medical instrument as set forth in claim 9 wherein the retention structure (76A, 78A) is adapted to signal that inter-engagement of the rotary connector (74, 76) and mounting structure (12) by the retention structure has been achieved.

11. A medical instrument as set forth in claim 10 wherein the retention structure (76A, 78A) is constructed for snap interengagement of the rotary connector (74, 76) and mounting structure (12) to signal the interengagement.

12. A medical instrument as set forth in any one of claims 9 to 11 wherein the retention structure comprises a first element (76A) association with the rotary connector (74, 76) and a second element (78A) associated with the mounting structure (12).

13. A medical instrument as set forth in claim 12 wherein the rotary connector further comprises a pair of arms (74) extending from the operative member (16) and a finger (76) on a free end of each arm, the mounting structure (12) including a slot (78) for each finger sized and shaped to receive the finger, the first element (76A) of the retention structure being associated with the finger and the second element (78A) being associated with the slot.

14. A medical instrument as set forth in claim 13 wherein the first element is a recess (76A) and the second element is a slot (78A).

15. A medical instrument as set forth in any one of claims 9 to 11 wherein the rotary connector (74, 76) and operative member (16) are formed as one piece.

16. A method of shielding a sharp end of a needle (14) of a needle assembly (10), the method comprising the steps of:
rotating a connector (74, 76) attaching a safety shield (16) to the needle assembly about an axis of the needle less than a full turn thereby to release the connection of the safety shield to the needle assembly;
sliding the safety shield along a longitudinal axis (LA) of the needle to a position in which the safety shield covers the sharp end (28, 32) of the needle.

17. A method as set forth in claim 16 wherein the step of rotating the connector (74, 76) comprises rotating the connector about the longitudinal axis (LA) of the needle less than 180 degrees to release connection of the safety shield (16) to the needle assembly (10).

18. A method as set forth in claim 17 wherein the step of rotating the connector (74, 76) comprises rotating the connector about the longitudinal axis (LA) of the needle (LA) about 90 degrees to release connection of the safety shield (16) to the needle assembly (10).
